Europäisches Patentamt

European Patent Office

Office européen des brevets

(11) Publication number: **0 109 072**
**B1**

# (12) EUROPEAN PATENT SPECIFICATION

(45) Date of publication of patent specification: **10.08.88**

(21) Application number: **83111298.2**

(22) Date of filing: **11.11.83**

(51) Int. Cl.⁴: **A 61 K 49/02, C 07 H 23/00,**
**C 07 H 3/04, C 07 B 59/00**

(54) **Diagnostic procedures using radio labeled sucralfate and derivatives or precursors thereof.**

(30) Priority: **12.11.82 US 441211**
**11.04.83 US 483757**

(43) Date of publication of application:
**23.05.84 Bulletin 84/21**

(45) Publication of the grant of the patent:
**10.08.88 Bulletin 88/32**

(84) Designated Contracting States:
**AT BE CH DE FR GB IT LI LU NL SE**

(56) References cited:
ARZNEIMITTEL FORSCHUNG, Vol. 30, no. 1,
January 1980 R. NAGASHIMA et al.: "Selective
binding of sucralfate to ulcer lesion.II.
Experiments in rats with gastric ulcer receiving
C-sucralfate or potassium C-sucrose sulfate",
pages 84-88.
CHEMICAL ABSTRACTS, Vol. 97, no. 15,
October 11, 1982, page 51, ref. 120364w
Columbus, Ohio, US Y. HINORARA et al.:
"Effects of Sucralfate on the permeability of
gastric mucosa to iodine-125-labeled human
serum albumin in experimental gastritis
induced by ethanol"

(73) Proprietor: **THE REGENTS OF THE UNIVERSITY**
**OF CALIFORNIA**
**2199 Addison Street**
**Berkeley California 94720 (US)**

(72) Inventor: **Vasquez, Tony E.**
**4343 E. Rialto**
**Fresno Cal. 92715 (US)**
Inventor: **Bridges, Robert L.**
**1652 Persimmon St.**
**Corona, Cal. 91720 (US)**
Inventor: **Braunstein, Philip**
**9 Fernbank**
**Irvine, Cal. 92714 (US)**
Inventor: **Jansholt, Anne-Line**
**16 Driftwood**
**Irvine, Cal. 92714 (US)**

(74) Representative: **Weickmann, Heinrich, Dipl.-Ing.**
**et al**
**Patentanwälte Dipl.-Ing. H.Weickmann Dipl.-**
**Phys.Dr. K.Fincke Dipl.-Ing. F.A.Weickmann**
**Dipl.-Chem. B. Huber Dr.-Ing. H. Liska Dipl.-**
**Phys.Dr. J. Prechtel Postfach 860820**
**D-8000 München 86 (DE)**

Courier Press, Leamington Spa, England.

(56) References cited:
**CHEMICAL ABSTRACTS, Vol. 99, no. 7 August 15, 1983, page 221, ref. 49703q Columbus, Ohio, US T.E. VASQUEZ et al.: "Work in progress. Gastrointestinal ulcerations: detection using a technetium-99m-labeled ulcer-avid agent"**

## Description

### Background of the invention

A new Food and Drug Administration approved drug, Sucralfate, for the short-term treatment of upper gastrointestinal ulcers, has been proven to bind selectively to ulcerated areas in the upper G.I. tract. Several clinical trials have shown the safety and efficacy of Sucralfate in healing duodenal ulcers.

Radiologic studies are preferred by many as the first diagnostic procedure when intestinal disease is suspected. They are reasonably accurate in detecting ulcer disease in most instances. Radiologic studies are more widely available and are noninvasive safer and less expensive then endoscopy, *Applied Radiology*, May/June 1982, pp. 20 and 120.

It is known that sucralfate binds to duodenal and gastric ulcers and to gastric erosions produced by ethanol and anti-inflammatory drugs. The affinity of sucralfate for defective mucosa is explained by the drug's viscous adhesiveness and the formation of polyvalent bridges between the negatively charged sucralfate polyanions and positively charged proteins present in high concentrations in mucosal lesions. Sucralfate also buffers acid, inhibits the action of pepsin, and adsorbs bile salts. These properties of sucralfate enable the drug to act as an effective barrier to the penetration of acid, pepsin, and bile salts. Sucralfate also binds to uninjured mucosa to a much lesser extent and is believed to exert a similar "barrier" effect on regenerated and normal mucosa. Other possible mechanisms for sucralfate's antiulcer effect include depletion of, acid, pepsin, and bile salts from the gastric secretion. Animal data show that the action of sucralfate is sustained because of its viscous adhesiveness, slow reaction with acid, and high affinity for areas with defectice mucosa, J. Clin. Gastroenterol 2 (supp 2): 117—127, 1981.

Studies involving $^{14}$C-labeled sucralfate in rats using histoautoradiographic methods have also been reported, Duodenal Ulcer Gastric Ulcer, Sucralfate, A New Therapeutic Concept, edited by Wolfgang F. Caspary, Hanau, West Germany, published by Urban and Schwarzenberg, Munchen-Wien-Baltimore, 1981, pp 19—21. However, $^{14}$C-labeled sucralfate is unsuited for in vivo imaging in humans.

We have developed a simple method for labeling Sucralfate with Tc-99m and other radio labels. The resulting suspension is easily administered orally and imaging may be carried out with standard scintigraphic equipment. Preliminary animal and human studies show that the agent is stable in vivo and has clinical utility for the evaluation of gastrointestinal ulcer disease along with other diseases that are associated with loss of mucosal integrity.

The method is simple, should have ready patient acceptability and be associated with low radiation doses. It is believed that the present invention presents a significant advance in the art.

The invention provides a novel radiopharmaceutical composition useful for the diagnostic procedure for the in vivo clinical evaluation of gastrointestinal ulcer disease and other diseases associated with loss of mucosal integrity in humans which comprises the oral administration of an effective amount of radio-labeled sucralfate or derivative or precursor thereof to the human host, followed by scintigraphic imaging of the gastrointestinal area of interest with scintigraphic imaging equipment.

The novel radiopharmaceutical composition comprises an aqueous solution or suspension containing an amount of a radio-labeled sucralfate or derivative or precursor thereof effective for in vivo scintigraphic imaging of the gastrointestinal or other mucosal areas in humans.

### Summary of the invention

The present invention is useful for a novel diagnostic procedure for the in vivo clinical evaluation of gastrointestinal ulcer disease and other diseases associated with loss of mucosal integrity in humans which comprises the oral administration of an effective amount of radiolabeled sucralfate or derivative thereof to the human host, followed by scintigraphic imaging of the gastrointestinal area of interest with scintigraphic imaging equipment.

The invention comprises radiolabeled sucralfate or derivative or precursors thereof wherein the radiolabel is one suitable for in vivo imaging in humans.

The invention still further comprises a novel radiopharmaceutical composition of an aqueous suspension or solution containing an amount of a sucralfate or derivative or precursors thereof labeled with a radioisotope which is a gamma emitter or position emitter effective for in vivo scintigraphic imaging of the gastrointestinal tract and other mucosal areas in humans.

It is an object of this invention to provide a new radioimaging technique for the in vivo detection of ulcers and related and associated diseases of the mucosa of the gastrointestinal tract.

These and other objects and advantages of our invention will be apparent from the more detailed discussion which follows.

### Description or the preferred embodiments

Technetium-99m labeled Sucralfate combines a short lived radioisotope which is most suitable for diagnostic imaging with a new oral medication which selectively binds to ulcers in the stomach and upper small bowel. This provides a safe, noninvasive and benign way to detect, localize and access for the presence and the activity of ulcer disease in the upper GI tract, both for initial diagnosis and follow-up evaluation.

The utility provided by our invention would replace or significantly decrease the need for the alternative methods of making a diagnosis which are relatively costly, less than adequately sensitive, often uncomfortable, and occasionally associated with significant risks.

There are no published data describing the use of radiotracer labeled Sucralfate or any other ulcer avid agent for the diagnosis of gastrointestinal ulcer disease.

The following Example is presented solely to illustrate the invention and should not be regarded as limiting in any way.

Example

1. 1—2 crushed Sucralfate tablets (1 gram per tablet were suspended in HCl at a pH of about 1 to 2.

2. 7—11×10¹⁰s⁻¹ (2—3 mCi) Technetium-99m labeled Human Serum Albumin were combined with the Sucralfate in the presence of stannous tartrate.

3. The resultant compound was washed with deionized water.

4. The drug was then administered orally in the form of a suspension or the product in water.

5. The gastrointestinal area of the patient was then imaged with a gamma camera.

Other modifications include tagging Sucralfate with other Technetium-99m compounds or other radioisotope labels useful for imaging. For example, I¹³¹ can be used. Positron emitters can be used in lieu of gamma emitters. It is also intended to use other human or non-human proteins or protein derivatives in place of human serum albumin to aid in radio labeling. The sucralfate can be replaced by derivatives thereof, preferably basic aluminum salts of a sulfated disaccaride, *per se*, a known class of compounds. The sucralfate can also be replaced by sucralfate precursors, for example, the sucrose moiety of sucralfate or related compounds, also a known class of chemical compounds.

The sucralfate or derivative or precursor thereof is preferably labeled under acidic conditions with radiolabled human serum albumin. However, amino acids, bovine albumin and other lower molecular weight proteins can also be used. The acidic conditions can be provided by HCl or any other mild acid.

The stannous tartrate in the Example acts as a reducing agent and other reducing agents will be useful for this purpose.

We anticipate based on our animal studies, that studies with this agent will be significantly more sensitive for the detection and localization, etc. of upper gastrointestinal ulcer disease than either of the only other two modalities available for this purpose, i.e., upper gastrointestinal barium x-ray series or endoscopy. We have been able to clearly identify ulcers in rabbits as small as 1 mm in size. These are most unlikely to be detected by the currently used methods. Furthermore, it should be associated with only a possible minimal risk from radiation, albeit lower in general than with upper GI series. It will also likely involve the patient only in minimal degree of discomfort and pyschological stress. Upper GI series and endoscopy are unpleasant, occasionally are associated with significant risk and/or require ability for agile cooperation on the part of the patients.

Initial studies in humans were conducted in three patients having confirmed ulcer disease. A one gram sucralfate tablet was first acidified and then labeled with 7—11×10¹⁰s⁻¹ (2—3 mCi) Technetium-99m labeled albumin and made into an aqueous suspension. A small amount of an inert binder was also present. The acid was washed away leaving a slurry of fine particles which were then mixed with about 5 ml of water and administered orally by swallowing. Imaging showed clearly that the labeled drug adhered selectively to the ulcerated portion of the mucosa. These areas were apparent.

It is estimated that 10% of the U.S. population has upper gastrointestinal ulcer disease. Most of these patients will undergo either one or both of the presently available studies, i.e. endoscopy or upper gastrointestinal x-rays repeatedly. In addition, a large number of patients who do not have ulcer disease undergo these examinations in order to rule such disease out.

The radiolabel is present in an effective amount per dose which is usually on the order of 4 to 7 or more up to 37×10¹⁰s⁻¹ or even more.

Having fully described the invention, it is intended that it be limited only by the lawful scope of the appended claims.

**Claims**

1. A radiopharmaceutical composition comprising an aqueous solution or suspension containing an amount of a sucralfate or derivative or precursor thereof, labeled with a radioisotope which is a gamma emitter or positron emitter for use in in vivo scintigraphic imaging of the gastrointestinal mucosal areas in humans.

2. The composition of Claim 1 wherein the radiolabeled sucralfate or derivative or precursor thereof is provided with label by complexing said sucralfate or a derivative or precursor thereof with ablumin or other protein or protein derivative labeled with a radioisotope which is a gamma or positron emitter under acidic conditions.

3. The composition of Claim 1 wherein the radiolabeled sucralfate or derivative or precursor thereof is provided with label by complexing said sucralfate or a derivative or precursor thereof with albumin or other protein derivative labeled with Technetium-99m radioisotope under acidic conditions and in the presence of a reducing agent.

4. The composition of Claim 1 wherein the sucralfate derivatives are basic aluminum salts.

5. The composition of Claim 1 wherein the sucralfate precursor is the sucrose moiety of sucralfate.

6. The composition of Claim 3 wherein the reducing agent is stannous tartrate.

7. Radiolabeled sucralfate or derivative or precursor thereof wherein the radiolabel is a radioisotope which is a gamma or positron emitter.

**Patentansprüche**

1. Radioaktive pharmazeutische Zusammensetzung, umfassend eine wäßrige Lösung oder Suspension mit einem Gehalt in Sucralfat oder dessen Derivate oder Vorstufen davon, das mit einem Radioisotop markiert ist, welches ein Gammastrahler oder Positronstrahler ist und die zur invivo-Abbildung mittels Szintigrafie der gastrointestinalen Schleimhautbereiche am Menschen angewendet wird.

2. Zusammensetzung nach Anspruch 1, in der das radioaktiv markierte Sucralfat oder dessen Derivate oder Vorstufen davon dadurch mit einem Marker versehen wird, daß das Sucralfat oder seine Derivate oder Vorstufen davon mit Albumin oder einem anderen Protein oder Proteinderivat, das mit einem Radioisotop markiert ist, welches ein Gamma- oder Positronstrahler ist, unter sauren Bedingungen komplexiert wird.

3. Zusammensetzung nach Anspruch 1, in welcher das radioaktiv markierte Sucralfat oder dessen Derivate oder Vorstufen davon dadurch mit einem Marker versehen wird, daß das Sucralfat oder eines seiner Derivate oder Vorstufen mit Albumin oder einem anderen Proteinderivat, das mit einem Technetium-99m-Radioisotop unter sauren Bedingungen und im Beisein eines reduzierenden Mittels komplexiert wird.

4. Zusammensetzung nach Anspruch 1, in der die Sucralfatderivate basische Aluminiumsalze sind.

5. Zusammensetzung nach Anspruch 1, in der die Sucralfatvorstufe der Sucrose-Teil des Sucralfats ist.

6. Zusammensetzung nach Anspruch 3, in der das reduzierende Mittel Zinntartrat ist.

7. Radioaktiv markiertes Sucralfat oder dessen Derivat oder Vorstufe indem der radioaktive Marker ein Radioisotop ist, der ein Gamma- oder Positronstrahler ist.

**Revendications**

1. Composition radiopharmaceutique comprenant une solution ou une suspension aqueuse contenant une quantité d'un sucralphate ou dérivé ou précurseur de celui-ci, marqué par un radio-isotope qui est un émetteur de rayons gamma ou un émetteur de positrons en vue de l'utilisation en imagerie scintigraphique in vivo des zones gastro-intestinales muqueuses chez l'homme.

2. Composition selon la revendication 1, dans laquelle on dote le sucralphate radiomarqué ou son dérivé ou précurseur d'un marqueur, en complexant ledit sucralphate ou un dérivé ou précurseur de celui-ci avec de l'albumine ou une autre protéine ou un dérive de protéine marqué à l'aide d'un radio-isotope qui est un émetteur de rayons gamma ou de positrons dans des conditions acides.

3. Composition selon la revendication 1, dans laquelle on dote le sucralphate radiomarqué ou dérivé ou précurseur de celui-ci d'un marqueur en complexant ledit sucralphate ou un dérivé ou précurseur de celui-ci avec de l'albumine ou autre dérivé de proteine marqué par radio-isotope de technetium-99m dans des conditions acides et en présence d'un agent réducteur.

4. Composition selon la revendication 1, dans laquelle les dérivés de sucralphate sont des sels d'aluminium basiques.

5. Composition selon la revendication 1, dans laquelle le précurseur de sucralphate est constitué par la fraction sucrose du sucralphate.

6. Composition selon la revendication 3, dans laquelle l'agent réducteur est du tartrate stanneux.

7. Sucralphate radiomarqué ou dérivé ou précurseur de celui-ci, dans lequel le radiomarqueur est un radio-isotope, lequel est un émetteur de rayons gamma ou de positrons.